Europäisches Patentamt

European Patent Office

Office européen des brevets

(19)

(11) Veröffentlichungsnummer : **0 204 908**
**B1**

(12)    EUROPÄISCHE PATENTSCHRIFT

(45) Veröffentlichungstag der Patentschrift :
**01.02.89**

(21) Anmeldenummer : **86104345.3**

(22) Anmeldetag : **29.03.86**

(51) Int. Cl.⁴ : **A 61 M 35/00**

(54) Verfahren und Vorrichtung zur Beaufschlagung von Augenstäbchen mit Wirkstofflösungen oder -suspensionen.

(30) Priorität : **13.04.85 DE 3513288**

(43) Veröffentlichungstag der Anmeldung :
**17.12.86 Patentblatt 86/51**

(45) Bekanntmachung des Hinweises auf die Patenterteilung : **01.02.89 Patentblatt 89/05**

(84) Benannte Vertragsstaaten :
**AT BE CH DE FR GB IT LI LU NL SE**

(56) Entgegenhaltungen :
**DE--A-- 2 441 191**
**FR--A-- 2 529 789**
**GB--A-- 2 097 680**
**US--A-- 3 948 265**

(73) Patentinhaber : **Dr. Karl Thomae GmbH**
**Postfach 1755**
**D-7950 Biberach (Riss) (DE)**

(72) Erfinder : **Schilk, Leonhard**
**Wetterkreuzstrasse 23**
**D-7950 Biberach 1 (DE)**

## Beschreibung

Die Erfindung betrifft ein Verfahren und eine Vorrichtung zur Beaufschlagung von Augenstäbchen mit Wirkstofflösungen oder -suspensionen. Die Augenstäbchen weisen hierbei an ihrem äußeren Ende rundumlaufend eine Wirkstoffschicht mit definiertem Wirkstoffgehalt auf. Bei den Augenstäbchen handelt es sich um Wirkstoffträger zur Applikation am Auge. Die vorzugsweise am äußeren Ende eines Stäbchens aufgebrachte Wirkstoffmenge läßt sich dadurch praktisch quantitativ in den Bindehautsack des unteren Augenlids einbringen, daß das Stäbchen zwischen Auge und Unterlid eingeschoben und um seine Achse gedreht wird.

Durch die deutsche Patentschrift Nr. 2 441 191 sind Augenstäbchen bekannt, die dadurch gekennzeichnet sind, daß eine für das Auge bestimmte aktive Substanz auf der Oberfläche des Trägerendes bzw. der Trägerenden in einer sehr dünnen, gleichmäßigen, trockenen und flüssigkeitslöslichen Schicht in einer derartigen Menge, wie sie einer Einzeldosis für die spezifische Gebrauchsanwendung entspricht, angeordnet ist. Das Aufbringen der aktiven Substanz auf den Träger erfolgte bisher durch ein gesteuertes Eintauchen der Enden der Träger in eine Lösung der Wirksubstanz oder durch Aufsprühen der Substanz auf den Träger oder durch Anbringen einer getrockneten aktiven Substanz mit Hilfe eines Klebers auf dem Träger. Es zeigte sich in der Praxis, daß das Aufbringen des Wirkstoffes nach den dort beschriebenen Methoden mit den folgenden Mängeln behaftet war : die Dosierung war zu ungenau ; die flächenbezogene Wirkstoffmenge wies größere Unterschiede auf ; bei einem Aufsprühen entstanden Tröpfchen von unterschiedlichem Durchmesser, es kam dabei zu einer teilweisen Vernebelung des Sprühgutes und damit zu einer ungleichmäßigen Beaufschlagung der dafür vorgesehenen Zonen, wobei andere Zonen des Trägers die nicht für eine Beaufschlagung vorgesehen waren, ebenfalls mit einem Wirkstoff-Film beschichtet wurden. Ein Aufstäuben des getrockneten Wirkstoffes bei Vorhandensein eines Klebers an den Stäbchen führte ebenfalls zu großen Wirkstoffschwankungen entlang den zur Beaufschlagung vorgesehenen Zonen des Trägers.

Es wurde nun gefunden, daß sich diese Nachteile dann vermeiden lassen, wenn Wirkstofflösungen oder Wirkstoffsuspensionen in Form von Tröpfchen gleichen Volumens mittels einer Mikrodosiervorrichtung, die pro Träger immer die gleiche Anzahl an Tröpfchen abgibt, auf den dafür vorgesehenen Zonen eines rotierenden Trägers aufgebracht werden. Die Rotation des Trägers besorgt eine gleichmäßige Rundumverteilung der so aufgebrachten Wirkstofflösungen oder -suspensionen auf den dafür vorgesehenen Zonen. Gleichzeitiges oder nachgeschaltetes Verdunsten des Lösungs- oder Suspensionsmittels und Trocknen des als Rückstand verbleibenden Wirkstoffes durch geeignete Verdunstungs- bzw. Trocknungsmethoden bewirkt ein Anhaften des Wirkstoffes auf dem Träger.

Bei diesem Verfahren des Beaufschlagens der Träger mit Wirkstoffen ergeben sich die folgenden Vorteile : entsprechend der Genauigkeit des Mikrodosiersystems läßt sich eine hohe Dosiergenauigkeit erzielen ; auf den zur Beaufschlagung vorgesehenen Zonen des Trägers erfolgt eine gleichmäßige Wirkstoffverteilung ; da die Wirkstofflösung oder -suspension in einem geschlossenen System gehalten werden kann, wird die Kontaminationsgefahr sehr stark vermindert ; ein Verdunsten des Lösungsmittels und damit eine Konzentrationsverschiebung des Wirkstoffes im Lösungsmittel wird verhindert ; der Wirkstoffvorrat läßt sich vollkommen aufbrauchen, es verbleiben keine nennenswerten Restmengen ; es lassen sich Mehrfachbeschichtungen durchführen, womit auch die Herstellung von Kombinationspräparaten ermöglicht wird.

Das Beaufschlagen bestimmter Zonen des stäbchenförmigen Trägers wird mittels eines kombinierten Rotations- und Transportsystems, welches ein weiterer Gegenstand der vorliegenden Erfindung ist, durchgeführt. Hierzu eignet sich z. B. ein gestrecktes Rotations- und Transportsystem aber auch ein rundes Rotations- und Transportsystem, bestehend aus einem sogenannten Transportrad in Verbindung mit einem Rotationsband. Im folgenden werden beide Systeme beschrieben, die aber beide dem gleichen Funktionsprinzip dienen : Beladen der Stäbchen durch Mikrodosierung, gleichmäßige Wirkstoff-Rundumverteilung und Trocknung durch Rotation der Stäbchen im Warmluftstrom oder mittels anderer geeigneter Trocknungsverfahren, z. B. mit IR-Strahlen- oder Hochfrequenztrocknern.

A. Gestrecktes Rotations- und Transportsystem.

Beschreibung der Funktonsweise an Hand der Figuren I bis III :

Legt man mehrere parallel angeordnete Stäbchen (8) auf eine ebene Unterlage und preßt diese gegen die Unterseite eines angetriebenen Rotationsbandes (1), so werden die Stäbchen in Richtung der sie antreibenden Bandseite transportiert (siehe Figur I). Nimmt man als Unterlage anstatt einer ruhenden Fläche die Oberseite eines zweiten Transportbandes (2), das in gleicher Drehrichtung mit gleicher Geschwindigkeit separat angetrieben wird, so werden die Stäbchen in eine Drehbewegung versetzt, ohne daß sie transportiert werden (siehe Figur II).

Läßt man die beiden Antriebsmotoren mit unterschiedlicher Geschwindigkeit laufen, so entsteht zur Rotationsbewegung eine Transportbewegung, deren Richtung vom schneller laufenden Band bestimmt wird (siehe Figur III).

In den Figuren I bis III ist (1) ein Rotationsband, (2) das gegenläufige Rotationsband, (1a) der Antrieb des Rotationsbandes (1), (1b) der Antrieb des Rotationsbandes (2) und (8) kennzeichnet die Stäbchen als Träger des Wirkstoffes.

Aufbau der Vorrichtung :

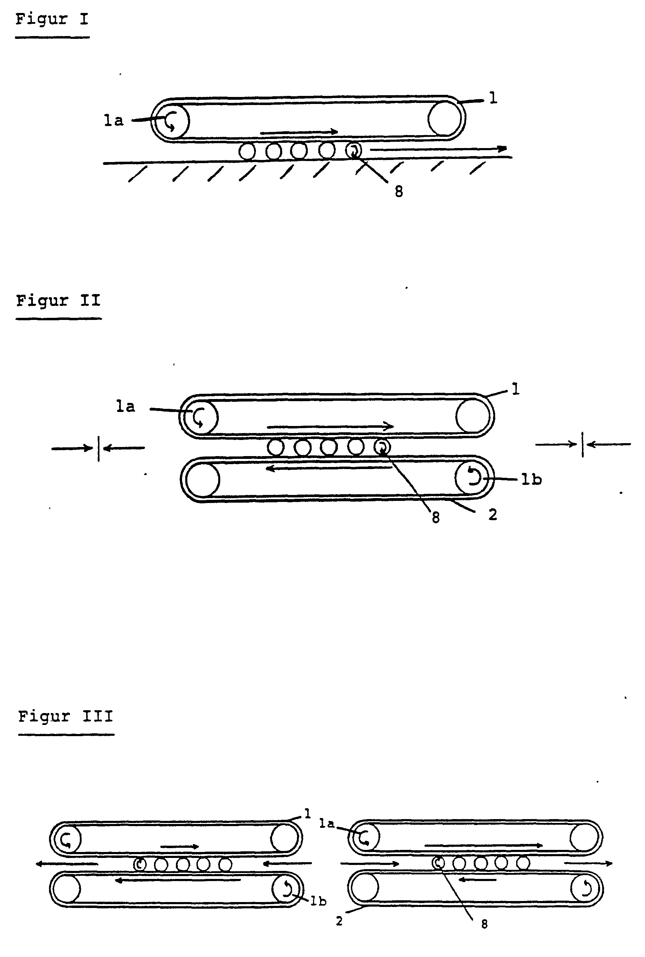

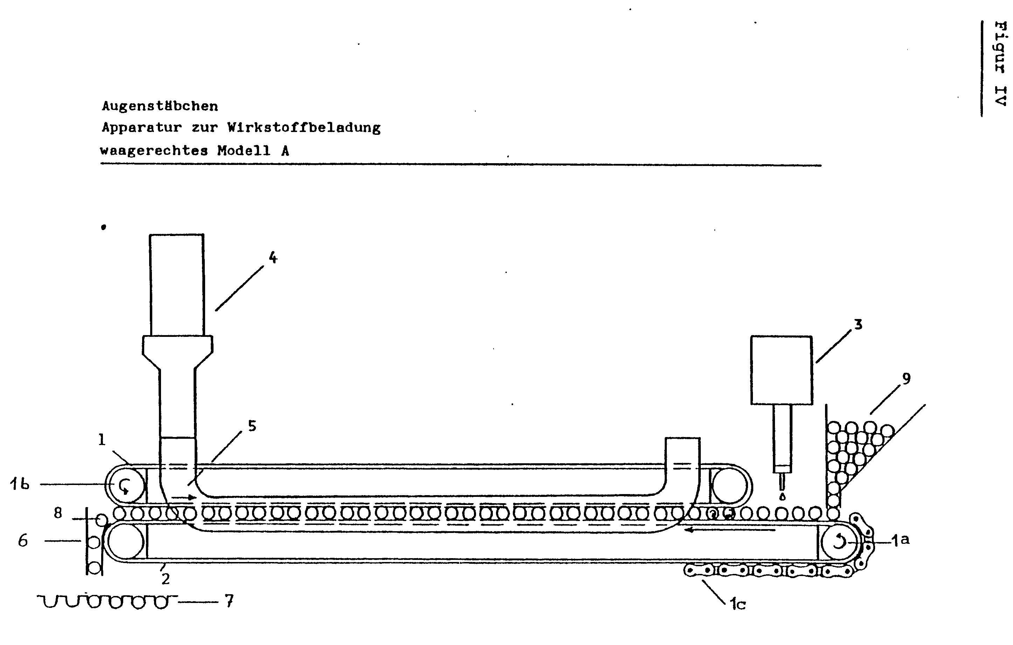

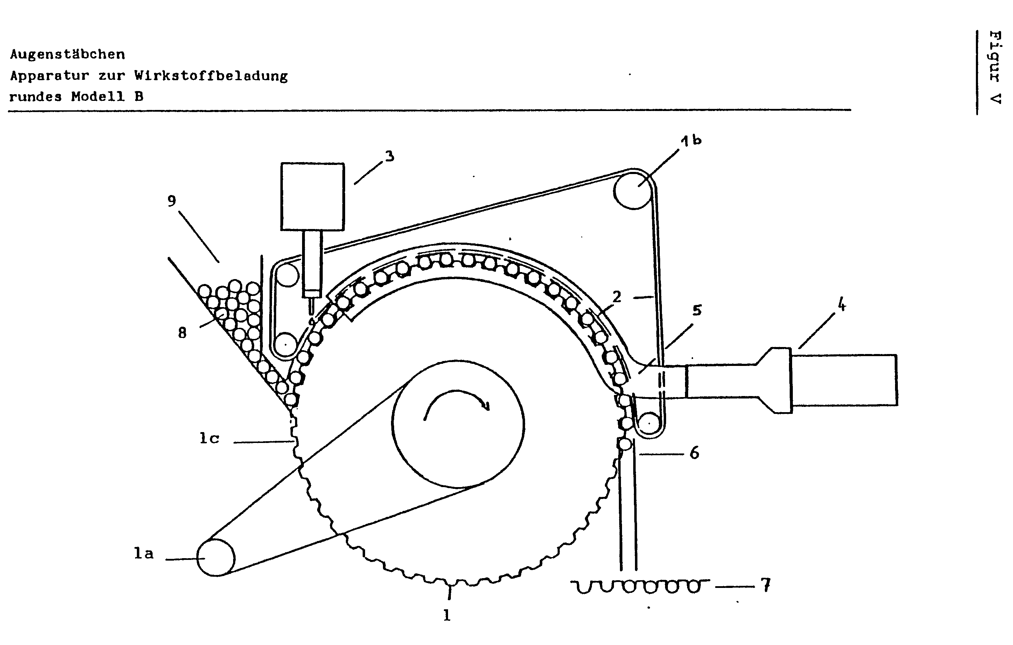

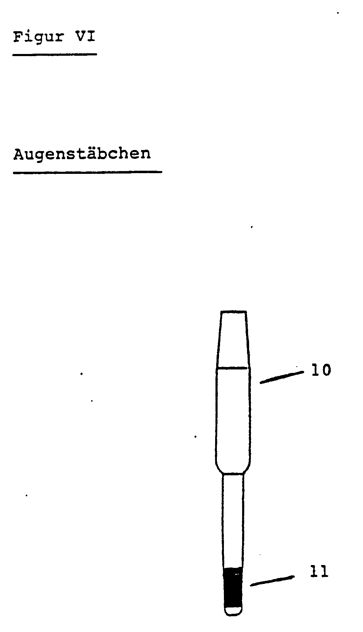

Der Aufbau der Vorrichtung wird durch die Figur IV in beispielhafter Weise verdeutlicht. Zwei horizontale Rotationsbänder (1) und (2) sind übereinander angeordnet. Jedes Band wird von einem separaten regelbaren Motor angetrieben (1a, 1b). Um das untere Band wird eine endlose Rollenkette (1c) zur parallelen Führung der Stäbchen gelegt. Der Hohlraum zwischen zwei Kettenquergliedern dient zur Aufnahme je eines Stäbchens und ist an die Form der Augenstäbchen angepaßt. Die Kettenquerglieder haben gleichzeitig die Funktion der Stäbchenmitnahme aus dem Vorratsbereich (9). Die Rollen der Kette werden ebenso wie die Stäbchen durch den Geschwindigkeitsunterschied der beiden Bänder angetrieben. Die Rotationsbänder sind schmäler als die Führungskette, so daß diese nur teilweise, nämlich im Bereich eines vorzugsweise zylindrischen Stäbchenhandgriffes, verdeckt ist. Der Teil der Kette, der die Stäbchenspitze umgibt, ist zur Wirkstoffbeladung und Trocknung frei zugänglich.

Die von der Kette mitgenommenen Stäbchen werden an der Dosiereinrichtung (3) mit Wirkstofflösung an der Stäbchenspitze durch Aufpunkten einer bestimmten Anzahl von Wirkstofflösungstropfen nach einer, z. B. photoelektrischen Kontaktauslösung beladen. Die gleichmäßige Rundumverteilung der Beladelösung erfolgt durch die Rotation der Stäbchen (8).

Das Trocknungsgebläse (4) versorgt den Trocknungstunnel (5) mit Warmluft. Dieser Trocknungstunnel besteht aus einem seitlich geschlitzten Rohr. Dieses ist so über den frei zugänglichen Teil der Führungskette gestülpt, daß die beladenen Stäbchenspitzen, von der Kette geführt, den Trocknungstunnel durchlaufen.

Der Warmluftstrom und die kontinuierliche Rotation der Stäbchen bewirken eine gleichmäßige Lösemittelverdunstung und eine daraus resultierende homogene Rundumverteilung des trockenen Wirkstoffes an der Stäbchenspitze.

Nach Verlassen des Trocknungstunnels werden die fertig beladenen Stäbchen von der Übergabestation (6) an die Verpackung (7) weitergegeben. Dort werden sie z. B. in Blisterpackungen eingesiegelt.

B. Rundes Rotations- und Transportsystem

Das Funktionsprinzip dieses runden Rotations- und Transportsystems ist im wesentlichen identisch mit der vorstehend beschriebenen waagerechten Anordnung : Beladen der Stäbchen durch Mikrodosierung und gleichmäßige Wirkstoff-Rundumverteilung und Trocknung durch Rotation der Stäbchen.

Der Aufbau unterscheidet sich vom waagerechten Modell durch das Stäbchentransport- und Rotationssystem und wird in beispielhafter Weise durch die Figur V wiedergegeben. In der Figur V ist (1) ein Transportrad mit einem Antrieb (1a) zum Transport der Stäbchen (8), (2) ist ein Rotationsband mit einem Antrieb (1b), welches die Rotation der Stäbchen bewirkt. Das Transportrad (1) weist außenseitig Kerben (1c) auf, die der äußeren Form der Augenstäbchen (8) angepaßt sind. (3) stellt eine Dosiereinrichtung für die Wirkstofflösung oder -suspension dar. (4) ist ein Trocknungsgebläse zur Lösungsmittelverdampfung und ist mit dem Trocknungstunnel (5) verbunden. Die Position (6) gibt den Auslauf und die Übergabe der mit Wirkstoff beaufschlagten Stäbchen (8) an die Verpackungsstation (7) wieder.

Ein großes Transportrad (1) wird von einem Antriebsmotor (1a) in eine langsame Drehbewegung versetzt. Gegen dieses Rad wird ein Rotationsband (2) zwischen der Eingabestation (9) und dem Auslauf (6) gepreßt, das von einem separaten Motor (1b) angetrieben wird. An der Außenseite des Transportrades sind Kerben (1c) eingearbeitet. Diese dienen zur Aufnahme und parallelen Positionierung der Augenstäbchen. Durch das Rotationsband werden die Stäbchen in den Kerben kontinuierlich gedreht.

Wirkstoffbeladung, Trocknung, Übergabe und Verpackung erfolgen in identischer Weise wie bei dem vorstehend beschriebenen gestreckten Rotations- und Transportsystem.

Die Figur VI zeigt ein bevorzugtes Modell eines Augenstäbchens im Querschnitt. Hierbei ist (10) ein Handgriff und (11) kennzeichnet die mit dem Wirkstoff beaufschlagte Zone.

Als Dosiereinrichtung eignen sich mit entsprechenden Düsen ausgestattete mechanisch, elektrisch bzw. elektronisch gesteuerte Pumpdosiersysteme, die eine Abgabe einer in einer Zeiteinheit gleichbleibenden Tropfenzahl gestatten, wobei die Größe bzw. das Volumen der Tropfen immer gleichbleibt. Als günstig erwiesen sich piezoelektrische Schwinger an oder in einer Kapillare mit einem düsenförmigen Austrittsende ; diese Schwinger sind im Prinzip aus der Ink-jet-Technologie bekannt. Es eignen sich aber auch durch z. B. Induktion gesteuerte Kleinventile in Verbindung mit entsprechend geformten Düsen.

**Patentansprüche**

1. Verfahren zur Beaufschlagung von Augenstäbchen mit Wirkstofflösungen oder -suspensionen mit nachträglicher Trocknung des auf den Augenstäbchen verbleibenden Wirkstoffes, dadurch gekennzeichnet, daß

a) Wirkstofflösungen oder -suspensionen in Form von Tröpfchen gleichen Volumens mit Hilfe einer Mikrodosiervorrichtung, die pro Augenstäbchen immer die gleiche Anzahl von Tröpfchen abgibt, auf den dafür vorgesehenen Zonen der rotierenden Augenstäbchen aufgebracht und

b) gleichzeitig oder nachgeschaltet das Lösungs- oder Suspensionsmittel durch Verdunstung entfernt wird.

2. Vorrichtung zur Beaufschlagung von Augenstäbchen mit Wirkstofflösungen oder -suspensionen in Verbindung mit einer Trocknungseinrichtung, dadurch gekennzeichnet, daß diese

a) aus einem Mikrodosiersystem und

b) aus einem dem Transport der Augenstäbchen dienenden und, in Kombination hierzu,

c) aus einem die Rotation der Augenstäbchen besorgenden gegenläufigen, geraden oder gekrümmten Band besteht.

3. Vorrichtung gemäß Anspruch 2, dadurch gekennzeichnet, daß diese gemäß Figur IV zwei horizontale übereinander angeordnete Rotationsbänder (1) und (2) mit separaten Antrieben (1a) und (1b), eine endlose Rollenkette (1c) mit an die Form der Augenstäbchen (8) angepaßten Hohlräumen zwischen den Kettenquergliedern, eine Dosiereinrichtung (3) ein Trocknungsgebläse (4) mit einem Trocknungstunnel (5), einen Vorratsbereich (9) und eine Übergabestation (6) umfaßt.

4. Vorrichtung gemäß Anspruch 3, dadurch gekennzeichnet, daß die Rotationsbänder (1) und (2) schmäler als die Rollenkette (1c) sind und der Trocknungstunnel (5) so über den frei zugänglichen Teil der Rollenkette (1c) gestülpt ist, daß die beladenen Stäbchenspitzen den Trocknungstunnel durchlaufen.

5. Vorrichtung gemäß Anspruch 2 gekennzeichnet, wie in Figur V wiedergegeben, durch ein Transportrad (1) mit Antrieb (1a), ein an dem Transportrad (1) anliegendes Rotationsband (2) mit Antrieb (1b), außenseitig am Transportrad (1) angebrachten Kerben (1c), die der Form der Augenstäbchen (8) angepaßt sind, eine Dosiereinrichtung (3), ein mit einem Trocknungstunnel (5) verbundenes Trocknungsgebläse (4), einen Auslauf (6) und eine Eingabestelle (9), wobei das Rotationsband (2) an dem Transportband (1) zwischen der Eingabestelle (9) und dem Auslauf (6) anliegt. ·

## Claims

1. Process for loading eye rods with solutions or suspensions of active substance, with subsequent drying of the active substance remaining on the eye rod, characterised in that

a) solutions or suspensions of active substance in the form of droplets of uniform volume are applied, by means of a micrometering device which always releases the same number of drops to each carrier, to the zones of a rotating eye rod intended for this purpose, and

b) the solvent or suspension agent is simultaneously or subsequently removed by evaporation.

2. Apparatus for loading eye rods with solutions or suspensions in combination with a drying apparatus, characterised in that this apparatus consists of

a) a micrometering device

b) a straight or curved belt serving to convey the eye rods and, in conjunction therewith,

c) a counter rotating straight or curved belt which ensures rotation of the eye rods.

3. Apparatus as claimed in claim 2, characterised in that it comprises, as shown in Figure VI, two horizontal conveyor belts (1) and (2) arranged one above the other with separate drives (1a) and (1b), an endless roller chain (1c) having spaces adapted to the shape of the eye rods (8) between the transverse chain links, a metering device (3), a drying blower (4) with a drying tunnel (5), a storage area (9) and a delivery station (6).

4. Apparatus as claimed in claim 3, characterised in that the conveyor belts (1) and (2) are narrower than the roller chain (1c) and the drying tunnel (5) is fitted over the freely accessible part of the roller chain (1c) in such a way that the charged rod tips pass through the drying tunnel.

5. Apparatus as claimed in claim 2, characterised, as shown in Figure V, by a conveyer wheel (1) with a drive (1a), a conveyor belt (2) abutting on the conveyor wheel (1) with a drive (1b), notches or recesses (1c) and adapted to the shape of the eye rods (8), a metering device (3), a drying blower (4) connected to a drying tunnel (5), an exit (6) and an input point (9), the conveyor belt (2) abutting on the conveyor belt (1) between the input point (9) and the exit (6).

## Revendications

1. Procédé de charge de bâtonnets oculaires avec des solutions ou des suspensions de principe actif, avec séchage ultérieur du principe actif restant sur les bâtonnets oculaires, caractérisé en ce que

a) des solutions ou suspensions de principe actif sous la forme de gouttelettes de volume égal sont appliquées à l'aide d'un dispositif de microdosage, qui délivre toujours le même nombre de gouttelettes par bâtonnet oculaire, sur les zones des bâtonnets oculaires en rotation prévues à cet effet et

b) le solvant ou l'agent de suspension est éliminé simultanément ou ultérieurement par évaporation.

2. Dispositif pour la charge de bâtonnets oculaires avec des solutions ou suspensions de principe actif en liaison avec une installation de séchage, caractérisé en ce que celui-ci se compose

a) d'un système de microdosage, et

b) d'un système servant au transport des bâtonnets oculaires, et, en combinaison avec celui-ci,

c) d'une bande droite ou recourbée assurant la rotation des bâtonnets oculaires.

3. Dispositif suivant la revendication 2, caractérisé en ce que celui-ci comprend, conformément à la figure IV, deux bandes tournantes (1) et (2) horizontales disposées l'une sur l'autre avec des entraînements séparés (1a) et (1b), une chaîne à rouleaux sans fin (1c) avec des espaces creux adaptés à la forme des bâtonnets oculaires (8) entre les éléments transversaux de la chaîne, un dispositif de dosage (3), un ventilateur de séchage (4) avec un tunnel de séchage (5), une région d'alimentation (9) et une station de déversement (6).

4. Dispositif suivant la revendication 3, caractérisé en ce que les bandes tournantes (1) et (2) sont plus étroites que la chaîne à rouleaux (1c) et en ce que le tunnel de séchage (5) est enfoncé sur la partie librement accessible de la chaîne à rouleaux (1c) de telle sorte que les pointes chargées des bâtonnets traversent le tunnel de séchage.

5. Dispositif suivant la revendication 2, caractérisé, comme le montre la figure V, par une roue de transport (1) avec un entraînement (1a), une bande tournante (2) avec son entraînement (1b), ajustée sur la roue de transport (1), des rainures (1c) pratiquées sur la roue de transport (1) du côté externe, lesquelles sont adaptées à la forme des bâtonnets oculaires (8), un dispositif de dosage (3), un ventilateur de séchage (4) relié à un tunnel de séchage (5), une sortie (6) et un poste d'introduction (9), la bande tournante (2) étant ajustée sur la bande de transport (1) entre le poste d'introduction (9) et la sortie (6).

Figur I
_____

Figur II
_____

Figur III
_____

EP 0 204 908 B1

Augenstäbchen

Apparatur zur Wirkstoffbeladung

waagerechtes Modell A

Figur V

Augenstäbchen
Apparatur zur Wirkstoffbeladung
rundes Modell B

Figur VI

Augenstäbchen

4